# EUROPEAN PATENT APPLICATION

(11) **EP 0 908 860 A1**
(43) Date of publication of application: **14.04.1999**
(21) Application number: 97500171.0
(22) Date of filing: 13.10.1997
(51) Int. Cl.: G08B 21/00

(54) **Device for sensing the opening of the eyelids and the initial sleeping phase**

(71) Applicant: Cebollas Y Derivados, S.L., 31500 Tudela, Navarra (ES); ADT ESPANA, S.L., 50008 Zaragoza (ES)
(72) Inventor: Bas Bayod, Francisco, 50008 Zaragoza (ES); Agud Miguel, Jose, 31500 Tuleda, Zaragoza (ES)
(74) Representative: Perez Bonal, Bernardo

(57) **Abstract**

Composed of a support or spectacles (5) comprising a module (1) with infrared signal transmission and reception means directed to the eye pits and an electronic circuit either incorporated or not to support (5) for processing the signals issued from module (1), which is fitted with an electronic transmission/reception module (6), a module (7) for sensing the movement of support (5), two modules (8) and (9) for sensing the opening of the eyelids, a module (10) for sensing the initial loss of consciousness, an electric warning module (11) and an acoustic warning module (12). It includes the use of analog and digital discrete components or a microcontroller (63) for the digital processing of the signals generated and for transmitting the signals sensing the initial sleeping phase.

## Description

### OBJECT OF THE INVENTION

This specification refers, as the name implies, to an improved device for detecting the opening of the eyelids and the initial sleeping phase, of the type used primarily to prevent falling asleep while driving a vehicle or in the course of protection or surveillance missions.

### BACKGROUND OF THE INVENTION

It is a well-known fact that one of the main causes behind traffic accidents involves loss of consciousness as a result of falling asleep in the driver's seat. This is particularly important considering that 5% of the population is afflicted by apnea, a sleeping disturbance which causes a person to fall asleep involuntarily. A recent report issued by the Spanish National Traffic Headquarters reveals that 13% of traffic accidents within the Spanish territory are caused by vehicles after the driver has fallen asleep.

The above reflects the importance of providing a device designed to avoid such risk conditions, thus preventing the loss of human lives and material resources.

Two such known devices have been developed which, although meeting the above requirements to a certain degree, apply indirect detection methods and therefore produce a relatively low cause-effect correlation.

The first of these detection devices operates on the basis of the pressure exerted by the driver's hands on the driving wheel, which in the first place involves modifying the vehicle by incorporating pressure sensors, and secondly allows for the possibility that the device may actually provide false data in that monitoring the pressure on the driving wheel is a relatively unreliable and indirect conscience-loss detection method.

Patent ES 9202389 provides an example of the use of a pressure sensor fitted inside the driving wheel of the vehicle and connected to an electric circuit which activates a sound and light equipment in the event that the driver's hands are separated from the wheel because of drowsiness.

The second of these known devices allows for detection in the event that the driver's head losses its verticality, based on the assumption that the driver, upon losing consciousness, relaxes the neck muscles and thus lowers his head. This method also fails to provide a large measure of reliability in detecting the actual loss of consciousness in view that lack of verticality of the head does not necessarily imply that the driver is asleep, and in any case this is a mechanical method that relies on each individual's morphology and is therefore difficult to assess.

### DESCRIPTION OF THE INVENTION

The system that is the object of the invention intends to solve the shortcomings of the above devices in that its detection method indicates in a precise manner the moment in which the driver actually enters an unconscious phase by emitting an error-free warning.

The system is based on the sensing of the open or closed position of the eyelids via infrared signals. The method shows a high correlation with the cause to be detected because obviously the eyes, save when blinking, are open if the user is conscious, and closed - irregularly and for extended periods - if the user is asleep.

The invention advocated is fitted with up to three pairs of sensors located in a supporting element such as a spectacle frame designed to hold one or two pairs of said sensors in a working position adjacent to or directed toward the eye pit or eye pits without interfering with the user's vision, hereunder referred to generically as SUPPORT or SPECTACLES, each pair of sensors comprising an infrared signal emitter LED and an infrared signal phototransistor detector, each photodiode emitting infrared light that is reflected in the corresponding eye pit and partly returns to the phototransistor, which receives and conveys it electrically to the sensing circuit. In the open position of the eyelid, the reflection of the cavity is smaller, so that light energy received by the phototransistor is also smaller. On closing the eyelid, the reflection increases and the phototransistor receives a greater amount of energy, which in turn increases the electric signal. The infrared signal emission LED power is harmless at the working distances at which the device operates.

The third LED/phototransistor may be positioned, as necessary, in an area of the support or spectacles directed toward a fixed point on the user's face that is not necessarily close to the eye pit or eye pits (e.g. the forehead), so that the signal generated becomes a function of the distance between the support or spectacles and said point. This pair is necessary if the device is required to discern at all times whether the increase in the signal received is the result of the closure of the eyelids or the movement of the support or spectacles by the user; should this LED/phototransistor pair not be present, an erroneous sensing of closed eyelids could occur.

Optionally, only one LED/phototransistor pair may be used to detect the closing of the eyelids, the pair being directed toward a single eye pit and the other eye remaining free. This arrangement reduces the cost of the electronic equipment while providing a similar level of protection. In a further embodiment of the system, and depending on the use given to the device, the sensor for detecting the movement of the spectacles may not be necessary at all.

The system as described comprises a support or spectacles overlapping the infrared signal sensor(s), and an electronic plate or hybrid microelectric sensing circuit, a full or partially integrated warning device being fitted in the support or spectacles and powered from the vehicle battery or some other power source connected to said infrared sensors. The electronic plate acts on the user warning unit which, if acoustic, activates a buzzer located near the external ear, and, if electric, relies on electrodes installed in the spectacle rods or in places providing sufficient skin contact to generate a slight electric current that produces a sensation sufficiently uncomfortable for the driver to recover full consciousness.

The device is also fitted with a logic consciousness loss warning output that may be connected to or utilized by other local external warning systems integrated in the vehicle proper; or remote system, the signal being conveyed to a control center through a radio frequency system such as a portable telephone.

This invention provides an advantage over known means in that it allows immediate detection of the position of the eyelids (open/closed) and its evolution in time, based on which the user's entry in the first sleeping phase is established. Once this is detected, the driver may be warned through the system to prevent loss of consciousness and thus avoid high risk situations involving not only himself but other involuntarily bystanders.

Another important advantage lies in the special utility of the invention, rendering it applicable to drivers of all nature of vehicles, security personnel, hospital personnel operating precision machinery, night watchmen, sentries in high risk posts and in general to all persons which - as a result of their assignments or for any other reason - are required to maintain a voluntary or forced attention and vigil through the use of local or remote external systems.

### DESCRIPTION OF THE DRAWINGS

In order to better understand the object of the present invention, the attached drawings show a referred practical embodiment thereof, in which drawings:

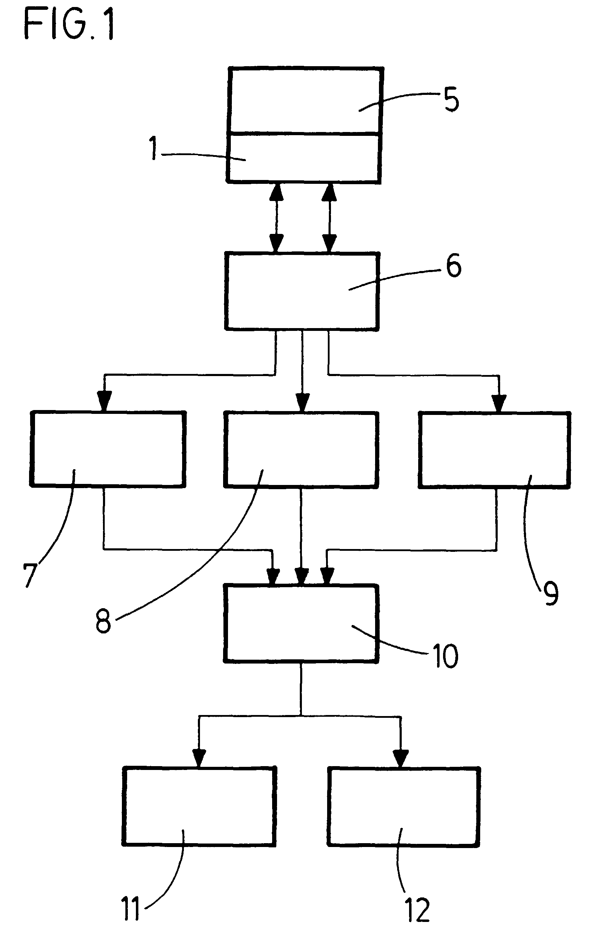

- Figure 1 shows an overall block diagram of the system that is the object of the invention.

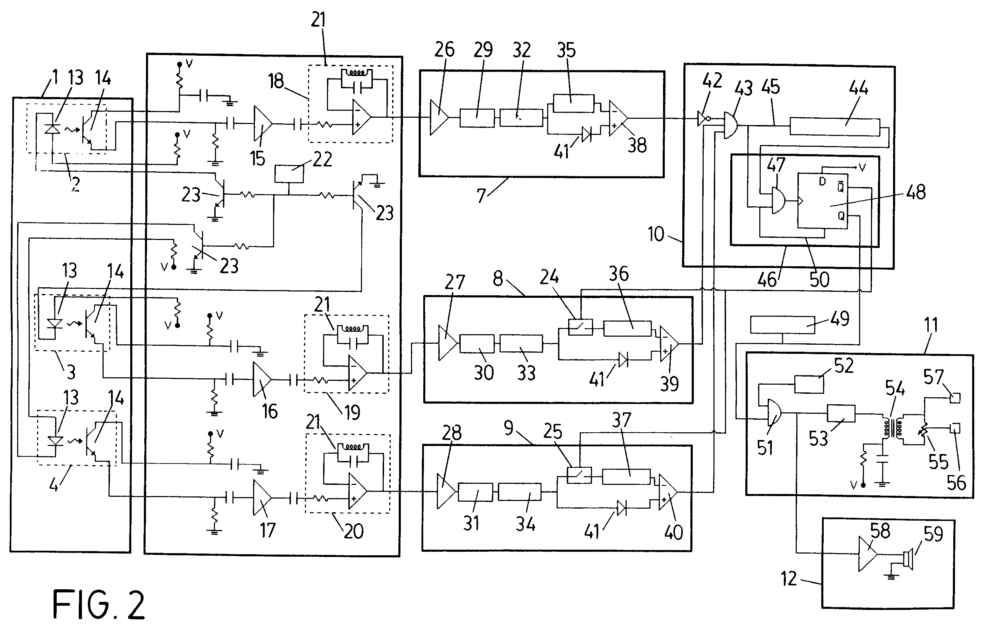

- Figure 2 shows a schematic diagram of all the modules forming the system, in an embodiment including discrete components.

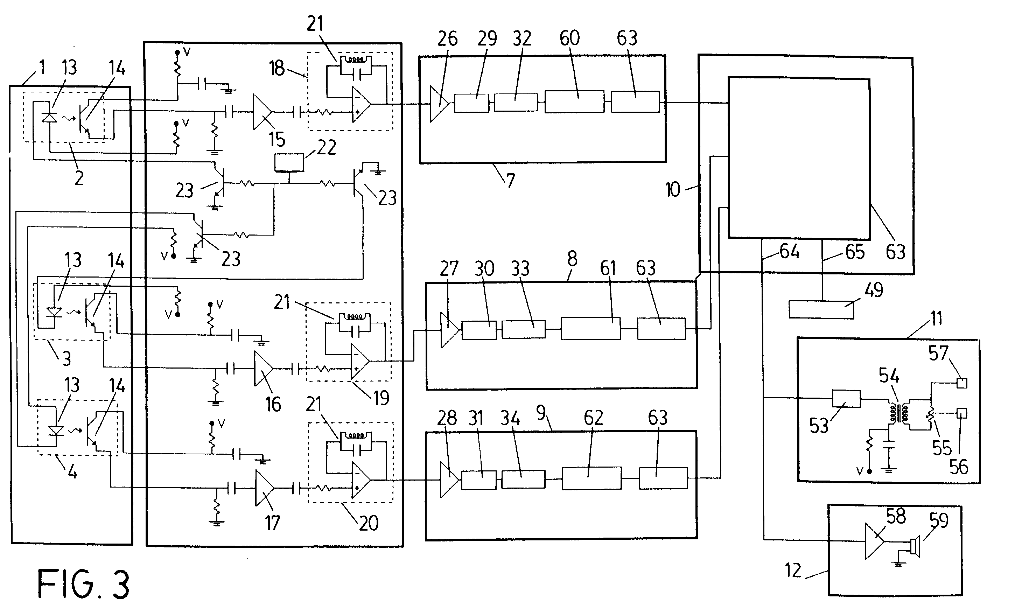

- Figure 3 shows a schematic diagram of all the modules forming the system, in an embodiment using a microcontroller.

### PREFERRED EMBODIMENT OF THE INVENTION

As can be seen from the above figures, the eyelid opening and initial sleeping phase detecting system that is the object of the present invention comprises a LED/phototransistor module (1) with infrared signal sensors (2), (3) and (4) fitted on a support or spectacles (5), a transmitter/receiver electronic module (6), a module (7) for sensing the movement of the spectacles (5), two eyelid position detecting modules (8) and (9), a loss of consciousness detecting module (10), an electric warning module (11) and an acoustic warning module (12). The functions of each module are described hereunder:

The infrared photodiodes (13) contained in the sensors (2), (3) and (4) and which are fitted in the support or spectacles (5) are activated through a 50 Khz square current wave with sufficient amplitude to generate a pulsing luminous power detectable by the phototransistors (14). Said phototransistors (14) in turn capture the radiation reflected in the eye pit or on the user's face, provided sensor (2) is used for detecting movement of the support or spectacles (5), converting the radiation in a pulsing electric signal with an amplitude in the order of tens of microamperes and a frequency of 50 Khz.

The transmitter/receiver module (6) captures these small pulsing electric signals from the phototransistors (14) and sends them on to the amplifiers (15), (16) and (17) fitted with filters, where they are filtered and their band width limited above and below the rated 50 Khz for subsequent amplification. They can optionally be filtered once again through bandpass filters (18), (19) and (20) built around the LC circuits (21) in order to further limit their band width to the 50 KHz central frequency range. The resulting signals are 50 Khz periodic signals with amplitudes in the order of volt units. This module (6) also pulse feeds the infrared signal diodes (13) via an oscillator (22) operating at a frequency of 50 Khz. The oscillator (22) generates a square signal in the above frequency which is applied, for each infrared signal diode (13), to the base of an NPN transistor (23) at the collector branch of which is located the corresponding infrared signal diode (13). This arrangement, whereby the transistors (23) periodically enter cutoff and conduction phases, sets off a pulsing current supply from the infrared signal diodes (13) resulting in a cyclic generation of luminous power.

The module (7) for detecting the movement of the support or spectacles (5) and the modules (8) and (9) for detecting the opening of the eyelids are identical, save for the analogic switch (24) and (25) fitted in the modules (8) and (9), and comprise an amplifier (26), (27) and (28), a rectifier circuit (29), (30) and (31), a filter (32), (33) and (34), a retainer circuit (35), (36) and (37) and a comparer (38), (39) and (40). The signals received from the transmitter/receiver module (6) relative to the eyelid opening and the position of the support or spectacles (5) are processed in said modules (7), (8) and (9). These periodic signals are first amplified at (26), (27) and (28); they are then rectified at (29), (30) and (31) to obtain positive unidirectional signals; they are subsequently filtered through filters (32, (33) and (34) to obtain a constant voltage, the value of which constantly acquires the amplitude value of the signals rectified at (29), (30) and (31) within a response time in the order of milliseconds; and are finally processed in the retainer circuit (35), (36) and (37) formed by an RC network designed to store the mean value of the amplitude of the previously rectified signal during a period of time that is a function of the circuit's response time, in the order of 10 seconds. The signals coming from the retainer circuits (35), (36) and (37) are conveyed to the inversion inputs of comparators (38), (39) and (40); the outputs from the filters reach the non-inversion inputs of said comparators (38), (39) and (40) through the diodes (41). The function of the diode (41) at the non-inversion input of the comparator (38), (39) and (40) consists in ensuring that, in stable or moving conditions of the eyeballs differing from the blinking motion, the diode output is a low level output, the corresponding comparator (38), (39) and (40) inversion input being kept at a value above that of the non-inversion input.

The analogic switch (24) and (25) remains closed until an initial loss of consciousness is actually detected, at which point it opens and is prevented from shutting until one of the eyelids opens.

When the eyelid closes, or the support or spectacles (5) comes close to the face, the non-inversion input signal of the corresponding comparators (38), (39) and/or (40) becomes larger than the inversion input signal as a result of the large response time of the retainer circuit (35), (36) and/or (37), the comparator (38), (39) and/or (40) output acquiring a high level and either triggering or - in the event of movement of the support or spectacles (5) - preventing the loss of consciousness detection process in module (10).

It must be borne in mind that, should the support or spectacles move toward the face, the outputs of the three comparators (38), (39) and (40) are set to their high level in view that the three signals coming from module (6) are enhanced, whereas in the event of closure of the eyelid only comparators (39) and (40) are affected.

During the time in which the eyelid remains closed, the signal at the comparator (39) and/or (40) inversion input slowly increases as a function of the retainer circuit (36) and/or (37) response time. Likewise, if the support or spectacles (5) come close to the face, the comparator (38), (39) and (40) inversion signal increases according to the retainer (35), (36) and (37) response time. In the latter case, the situation of the comparators (39) and (40) is irrelevant in view that consciousness loss sensing is inhibited during said transitional period, which period depends on the response time of the retainer circuit (35). This is due to the fact that once this time elapses, the signal at the comparator (38) inversion input reaches and exceeds the value at the non-inversion input, its output returning to its low level and disinhibiting the initial loss of consciousness sensing process. Response time is identical for the three retainer circuits (35), (36) and (37), so that, upon movement of the support or spectacles (5) toward the face, said three circuits (35), (36) and (37) reach their balance condition and thus prevent the loss of consciousness sensing process from being triggered.

If the eyelid opens or the support or spectacles (5) again move away from the face, the non-inverter input immediately acquires a voltage lower than that of the inverter input of the corresponding comparators (38), (39) and/or (40), their output acquiring a low level and thus deactivating the initial loss of consciousness sensing process. After this response time has elapsed, the output of the retainer circuits (35), (36) and/or (37) stabilizes at the eyelid opening value, or at the opening value of the new position of the support or spectacles (5). If the support or spectacles (5) move away from the face, the value of the three signals coming from module (6) diminishes, so that the outputs of the three comparators (38), (39) and (40) acquire their low level and the inversion inputs of said comparators (38), (39) and (40) go through a transitional period of a length approximately the same as that of the response time of retainer circuits (35), (36) and (37), without affecting the outputs.

During the winking motion, and because of the long response time of the retainer circuits (36) and (37), the value stored in the circuits reveals no appreciable change and a positive impulse takes place at the output of comparator (39) and/or (40), the duration of which coincides with the time during which the eye remains closed.

The module (7) for sensing the movement of the support or spectacles (5) is necessary because the amplitude value of the signals received by the modules that sense the eyelid (8) and (9) opening is affected, as previously stated, both by the opening and the closing of the eyelids and by the movement of the support or spectacles in respect to the face. The module (7) is therefore used for discerning at all times what the cause is for the variation of said signal.

In order to monitor only one eye pit, one infrared sensor (3) or (4) and associated processing circuits may optionally be removed.

The initial loss of consciousness sensing module (10) is formed by an inverter circuit (42) that receives the output of module (7) for sensing the movement of the support or spectacles (5) through an AND gate (43) which in turn receives the output from the previous inverter and from the eyelid opening sensing modules (8) and (9), via a timer (44) that receives in its RESET input (45) the signal coming from AND gate (43) and through a logic circuit (46) which senses the end of the timing (46), consisting in an AND gate (47) that receives the incoming timer RESET signal (45) and the timing end impulse, the output of which activates the input of a bistable unit (48). The output of said bistable unit (48) is accessed by other external warning systems which may be integrated in the vehicle proper, using the signal to start a warning process which could, for example, include the controlled automatic stopping of the vehicle or the transmission of said signal to a remote control center. The bistable unit (48) signal is conveyed to the exterior through a connector (49) provided for that purpose.

Under normal conditions, the eyelids remain open for extended periods of time, closing for short intervals when blinking. The comparator (39) and (40) must therefore be located at a low level, so that the AND gate output (43) will be 0 logic, affecting the RESET input (45) in timer (44), the count of which remains at zero, and the CLEAR input (50) of bistable unit (48), which remains erased.

If the support or spectacles (5) are in a stable condition or in a position away from the face, the module (7) output sensing the movement of the support or spectacles (5) remains at low level, thereby activating the relevant input from the AND gate (43). When the support or spectacles (5) leave their stable condition and move away from the face, the three outputs in module (6) decrease, so that module outputs (7), (8) and (9), and thus bistable unit (48), remain at a low level. As the support or spectacles (5) move toward the face, this is interpreted by the device as evidence that the user is initially losing consciousness, and the device will therefore ignore the events during the seconds subsequent to said movement. In this case, the output from module (7) will go on to a high level, the AND gate (43) output thus acquiring a low level and impairing the timing operation in view that the effect from module outputs (8) and (9) is annulled, despite the fact that they are both at a high level.

If the eyelids close during a stable condition, the AND gate (43) output is set to a 1 logic value, the outputs of modules (8) and (9) being set at a high level; timer (44) thus goes into operation. Timer (44) stops only when the output from AND gate (43) is 0 logic, a condition that takes place when the support or spectacles (5) draw near or when one of the eyelids opens. In the optional case wherein sensing occurs in only one of the eye pits, timing commences when the corresponding eyelid closes.

If the eyelids remain closed for a sufficiently long period of time, during which time AND gate (43) maintains a 1 logic value at the output, the timer (44) reaches the count producing a 1 logic value at the input of AND gate (47) in the logic circuit sensing the end of the timing (46) process. Since the output of AND gate (43) is set to 1 logic value, the output at gate AND (47) emits a positive impulse which activates bistable unit (48), so that the output acquires a 1 logic value.

This value 1 in bistable unit (48) deactivates the analogic switch (24) and (25) in modules (8) and (9), causing the retainer circuits (36) and (37) to preserve the stored value indefinitely, whereas bistable unit (48) remains activated. The result is therefore that the non-inversion input of comparators (39) and (40) is maintained indefinitely at a value above that of the inverter input, so that the output of modules (8) and (9) is kept at a high level, bistable unit (48) thus remaining activated while both eyelids are closed and becoming deactivated only in the event that one of them opens or that the support or spectacles (5) are drawn near the face, which in turn causes the analog switches (24) and (25) to close and the timer (44) value to be erased.

If the support or spectacles (5) are drawn away from the face while the eyelids are shut, the input signals in modules (8) and (9) for sensing the opening of the eyelids diminish; however, this reduction fails to compensate for the increase suffered by such signals on the closing of the eyelids, so therefore said module outputs remain at their high levels. This situation therefore allows the timer in the initial loss of consciousness sensing module (10) to continue, the corresponding warning being emitted if the eyelids remain closed for a specified period of time.

The electric warning module is formed by an oscillator (52) which generates a square frequency signal in the audio range of 500-3000 Hz. This signal is guided to an AND gate (51), to the other input of which is connected the bistable unit (48). The output from this gate leads to an exciter circuit (53) which acts on the primary circuit of a miniature transformer (54) in a 1:5 transforming ratio. The terminals of the secondary circuit in this transformer are connected to a potentiometer (55), the central terminal in said potentiometer forming the negative electrode (56) and one of the terminals in the secondary circuit forming the positive electrode (57), both electrodes being in contact with the skin.

The output in AND gate (51) is 0 logic while the bistable unit (48) remains at 0, providing a square signal of a frequency equal to that of the oscillator (52) upon activating said bistable unit. This means that, when the eyelids remain closed for a period larger than the timing period, the exciter circuit (53) activates the primary circuit in transformer (54) and generates an alternate current through the secondary circuit of transformer (54) and through the potentiometer (55), the electrodes (56) and (57) and finally the user's skin. Obviously, if the bistable unit (48) remains deactivated, no current is generated through the skin.

The acoustic warning module (12) uses the same signal generated at the AND gate (51), said signal being connected to the input of an amplifier (58) which finally activates a buzzer (59). Therefore, the square signal generated in the event that the eyelid remains closed for a sufficiently long period of time is amplified and conveyed directly to buzzer (59).

Modules (7), (8) and/or (9) may be partially implemented and module (10) totally implemented by a microcontroller (63) and applicable software. Said microcontroller (63) comprises at least one central processor unit with RAM memory, ROM memory, digital inputs/outputs, several timers and in-series and/or in-parallel communication gates. In this case, it is implemented discretely only in modules (7), (8) and (9), in amplifier circuits (26), (27) and/or (28), in rectifier circuits (29), (30) and/or (31), and in filters (32), (33) and/or (34). The function of the remaining components (retainer circuits (35), (36) and/or (37), comparators (38), (39) and/or (40), diodes (41) and analog switches (24) and/or (25)) is implemented internally in microcontroller (63), as is the function of all the module (10) components.

In order to introduce the values of the signals coming from filters (32), (33) and/or (34) in microcontroller (63), analog/digital converters (60), (61) and/or (62) are used. In this case, retainer circuits (35), (36) and/or (37) turn into mean calculation routines in second unit intervals applied over the digital signals coming from the analog/digital converters (60), (61) and/or (62) which generate S signals; the analog switches are converted into conditional jumps dependent on the value of the signal coming from module (7) for controlling the movement of the support or spectacles (5), said jumps inhibiting the sensing process when a movement is detected; and finally comparators (38), (39) and/or (40) and diodes (41) are converted into comparative instructions through the software of the mean values S and the instantaneous values supplied by the analog and/or digital converters (60), (61) and/or (62).

Thus, when a sufficiently high increment takes place in the instantaneous signal coming from the A/D converters (61) and/or (62) in respect to mean values S, without a variation of the digital signal coming from module (7) for detecting the movement of support or spectacles (5) taking place, a timer is activated which will not stop unless the signals from modules (8) or (9) diminish, the same effect taking place at the end of said timing period as the effect previously described for the system constructed on the basis of discrete components via the modules (11) and (12) by means of digital signal (64) from microcontroller (63), which generates a square wave to activate both modules (11) and (12). Digital signal (65) from microcontroller (63) is also activated, indicating to any external system connected to it that the initial sleeping phase has been reached. The mean signals S are calculated only when the support or spectacles (5) remain in repose, this being determined through module (7).

Microcontroller (63) may also be used for conducting a statistical processing of the signals coming from A/D converters (61) and/or (62) so that - with or without module (7) for detecting the movement of support or spectacles (5) - a more accurate sensing of the user somnolence condition can be achieved, since the signal coming from A/D converters (61) and/or (62) corresponds to a signal directly related to ocular dynamics and therefore represents, after simple manipulation, a stochastic process of an ergodic nature. Therefore, a power spectrum sampling of this input signal may be implemented in order to establish, as from this figure and through hypothesis testing, whether the samples lie within a certain normality or are openly abnormal. This anomalous sampling condition would indicate that the system's user status is unusual, implying, because of the high correlation between the ocular dynamics and the somnolence condition, that the driver is in an initial sleepiness condition and leading the system to act accordingly, as previously specified.

The use of a microcontroller in the embodiment of the device further allows a substantial cost and space saving, since the system may be implemented with the use of hybrid microelectronic circuits incorporated in the support or spectacles (5).

Operation of the system is as follows:

The user puts on the support or spectacles (5) fitted with infrared detectors (1). In this condition, two of the infrared sensors (3) and (4) are directed toward the eye pits, the other one (2) being directed toward a fixed point on the face. The warning electrodes (56) and (57), if available, are in contact with the skin; the buzzer (59) is close to one of the user's external ears. The monitoring of only one eyelid may optionally be performed, the other eyelid remaining free, or else this sensing may be conducted through a single sensor directed toward one of the eye pits.

The user turns the system on. At this time, the oscillator (22) powering the infrared photodiodes (13) starts its 50 Khz activity, whereby said photodiodes (13) emit light in a pulsing manner. This light is partly reflected and is then partly received in the phototransistors (14) and converted into electric signals to be amplified and filtered in transmitter/receiver module (6).

If discrete components are implemented, the signals coming from module (6) are conveyed to modules (7), (8) and (9) for sensing the support or spectacles (5) movement and the opening of the eyelids, the retainer circuits (35), (36) and (37) of which evolve according to their response time - approximately 10 seconds - until stable values are achieved.

In view that during this initial transition period the output from module (7) remains at a high level, timer (44) is deactivated and therefore no initial loss of consciousness is sensed. When retainer circuits (35), (36) and (37) reach their stable values, comparator (38), (39) and (40) outputs are set to their low value.

After this transition period, only the action of closing the eyelids, and no other, causes the timer (44) to be activated. If the eyelids remain closed for a period of approximately 1 second, the module (10) for detecting the loss of consciousness infers that the user of the support or spectacles (5) is into the first sleeping phase, in view of the fact that when timer (44) reaches the corresponding count during that period, the warning process - be it electric, acoustic or external - is activated. The bistable unit (48) remains on while the eyelids are closed, and is deactivated the moment one of the eyelids opens. As previously stated, even though the support or spectacles (5) may be drawn away from the face when the eyelids are closed, the device senses the user's initial loss of consciousness because the effect of the drawing away movement is smaller than that in the opposite sense as a result of the closing of the eyelids.

If the support or spectacles (5) come near the face, the timing process is inhibited for a time similar to the response time of retainer circuit (35) and the system interprets that the loss of consciousness phase is not going to start at least during that time interval. After this time has elapsed, the three retainer circuits (35), (36) and (37) should have reached their balance status, so that the start of the loss of consciousness sensing process proceeds as from the point it had been left off without causing undesirable effects.

The bistable unit (48) output may be connected to some other external local warning system or be conveyed to a remote control center using some kind of remote data transmission/reception system, such as a cellular telephone system, to enable full monitoring of the alert/attention condition of the person being controlled.

If the device monitors only one eye, the timing process starts when the relevant eyelid closes. The bistable unit (48) is activated if the eyelid remained closed for a period longer than the period marking the end of the timing process, and is deactivated when the eyelid opens.

If the system incorporates a microcontroller (63), the processing of the signals from the infrared sensors (1) is implemented as previously explained, save for the fact that the signals coming from the A/D converters (60), (61), and/or (62) are conveyed to microcontroller (63), where they are read as digital data. Microcontroller (63) incorporates, in the form of software, the same functions as the version fitted with discrete components. The signals coming from the A/D converters (61) and/or (62) are read and processed through the routines of retainer circuit (36) and/or (37) in order to obtain their S temporary mean values. Said mean values are compared with the instantaneous values specified for the A/D converters (61) and/or (62). When said instantaneous signals exceed the S mean values by a given margin, this indicates the start of the timing process, which is interrupted only in the event that movement of the support or spectacles is detected because of variations in the signal coming from the A/D converter (60) in module (7) for sensing movement of the support or spectacles, or in the event that one of the instantaneous signals coming from the A/D converters (61) and/or (62) falls below the calculated S mean values, thereby indicating that at least one eyelid is open. Once the timing process is under way, calculation of the S mean values stops, as in the case of the discrete components circuit, namely via the opening of the analog switches (24) and/or (25).

Once the closed eyelid sensing timing is completed, a warning process is triggered through modules (11) and (12) connected to one of the digital outputs (64) of microcontroller (63), designed to be activated when the initial loss of consciousness sensing process is over. This output is capable of generating a square frequency signal in the audio range of 500-3000 Hz, so that oscillator (52) may be obviated. Another digital output (65) from microcontroller (63) is conveyed to connector (49), thereby signaling the external system that the eyelid has remained closed for a specified period of time.

Other more elaborate closed eyelid and initial loss of consciousness sensing processes may be implemented through microcontroller (63), thus avoiding false detections with or without the use of module (7) for sensing the movement of support or spectacles (5). Statistical processing allows the initial sleeping phase to be sensed before the actual closing of the eyelid takes place. Specifically, signals coming from A/D converters (61) and/or (62) derive from a stochastic process which may be converted to ergodic, as previously stated. If such is the case, the signals coming from said A/D converters (61) and/or (62) are read at regular intervals and their temporary mean value calculated within a specified period. The difference between the signals read and the mean values calculated produces an ergodic process, so that, by processing said resulting signals, estimated values of the self-correlation function may be calculated. Based on the latter, sampled values estimated from the power spectrum of said signals may also be calculated. These estimated Pe(f) values, calculated periodically, are averaged in the course of a longer temporary interval and produce values near the actual P(f) values. Said P(f) values are then compared with the estimated Pe(f) values, and in the event that said Pe(f) estimated values differ considerably from the P(f) values, a warning process is activated through modules (11) and (12), as previously discussed, under the assumption that the user is in the initial sleeping phase; in this case, sensing of the closed eyelid becomes unnecessary.

Furthermore, in the embodiment based on microcontroller (63), the unit may be used for sensing lack of activity in the system, indicating that the user's eyelids are closed, or for sensing that the system is being used incorrectly or not at all. This circumstance may be alerted both internally, through modules (11) and (12), and externally through connector (49).

The nature of the invention and its form of embodiment being thus sufficiently described, we can only add that changes in form, materials, dimensions and arrangement may be implemented provided said alterations do not substantially alter the characteristics of the invention as claimed hereunder.

## Claims

1. Device for sensing the opening of the eyelids and the initial sleeping phase, of the type used for maintaining the user in a state of vigil, characteristic in that it comprises a support or spectacles incorporating a module (1) fitted with infrared signal transmission and reception means located near the eye pits and directed toward them without affecting the user's vision, and an electronic circuit either incorporated or not to said support or spectacles (5) for the purpose of processing the signals issued from module (1), which is fitted with a transmission/reception electronic module (6), a module (7) for sensing movement of the support or spectacles, two modules (8) and (9) for sensing the opening of the eyelids, a module (10) for sensing the initial loss of consciousness, an electric warning module (11) and an acoustic warning module (12).

2. Device for sensing the opening of the eyelids and the initial sleeping phase, according to the preceding claim, characteristic in that module (1) is fitted with a sensor (2) related to transmission/reception module (6) which in turn is related to module (7) for sensing movement of the support or spectacles, said sensor (2) comprising an infrared photodiode (13) and phototransistor (14), said module (1) also incorporating two sensors (3) and (4) equally related to module (6) for receiving data from module (1) and subsequently channeling it toward modules (8) and (9), said sensors (3) and (4) incorporating an infrared photodiode (13) and phototransistor (14).

3. Device for sensing the opening of the eyelids and the initial sleeping phase, according to the preceding claims, characteristic in that transmission/reception module (6) is fitted with bandpass filter amplifiers (15), (16) and (17), an oscillator (22) and NPN transistors (23).

4. Device for sensing the opening of the eyelids and the initial sleeping phase, according to the preceding claims, characteristic in that transmission/reception module (6) is fitted with bandpass filters (18), (19) and (20) which include LC circuits (21).

5. Device for sensing the opening of the eyelids and the initial sleeping phase, according to the preceding claims, characteristic in that module (7) for sensing movement of the support or spectacles (5) is associated at its input with module (6) and at its output with module (10), and in that it incorporates an amplifier (26), a rectifier circuit (29) and a filter (32).

6. Device for sensing the opening of the eyelids and the initial sleeping phase, according to the preceding claims, characteristic in that module (7) for sensing movement of the support or spectacles (5) incorporates, as from filter (32), a retainer circuit (35), a diode (41) and a comparator (38).

7. Device for sensing the opening of the eyelids and the initial sleeping phase, according to claims 1, 2, 4 and 5, characteristic in that module (8) for sensing the opening of the eyelids channels its data toward module (10) for sensing the initial loss of consciousness, said module (8) incorporating an amplifier (27), a rectifier (30) and a filter (33).

8. Device for sensing the opening of the eyelids and the initial sleeping phase, according to claims 1, 2 and 7, characteristic in that module (8) for sensing the opening of the eyelids incorporates, further along from filter (33), an analog switch (24), a retainer circuit (36), a comparator (39) and a diode (41).

9. Device for sensing the opening of the eyelids and the initial sleeping phase, according to claims 1 and 2, characteristic in that module (9) for sensing the opening of the eyelids channels its data toward module (10) for sensing the initial loss of consciousness and incorporates an amplifier (28), a rectifier circuit (31) and a filter (34).

10. Device for sensing the opening of the eyelids and the initial sleeping phase, according to claims 1, 2 and 9, characteristic in that module (9) for sensing the opening of the eyelids incorporates, further along from filter (34), an analog switch (25), a retainer circuit (37), a comparator (40) and a diode (41).

11. Device for sensing the opening of the eyelids and the initial sleeping phase, according to claims 1, 2, 5, 7 and 9, characteristic in that module (10) receives data associated to modules (7), (8) and (9), and is connected to electric warning module (11) and acoustic warning module (12), the latter incorporating an amplifier (58) and a buzzer (59).

12. Device for sensing the opening of the eyelids and the initial sleeping phase, according to claims 1, 2, 5, 7, 9 and 11, characteristic in that module (10) incorporates an inverter circuit (42), an AND gate (43), a timer (44) with the relevant RESET input (45) applicable to said timer (44), and a logic circuit for sensing the end of the timing period (46) which includes and AND gate (47) and a CLEAR input (50) corresponding to bistable unit (48).

13. Device for sensing the opening of the eyelids and the initial sleeping phase, according to claims 1 and 11, characteristic in that electric warning module (11) incorporates an exciter circuit (53), a transformer (54), a potentiometer (55) and contact electrodes (56) and (57).

14. Device for sensing the opening of the eyelids and the initial sleeping phase, according to claims 1, 11 and 13, characteristic in that electric warning module (11) initially incorporates, prior to exciter (53), an AND gate (51) and an oscillator (52).

15. Device for sensing the opening of the eyelids and the initial sleeping phase, according to claims 1, 5, 7, 9, 11 and 12, characteristic in that it incorporates a connector (49) at the output of module (10) which can be connected to an additional external warning system.

16. Device for sensing the opening of the eyelids and the initial sleeping phase, according to claims 1, 5, 7, 9, 11, 12 and 15, characteristic in that bistable unit (48) in module (10) is connected to connector (49).

17. Device for sensing the opening of the eyelids and the initial sleeping phase, according to claims 1 to 5, 7, 9, 11 and 13, characteristic in that it incorporates a microcontroller (63) formed by at least one central processing unit, RAM memory, ROM memory, digital inputs/outputs, several timers and in-series and/or in-parallel communication gates.

18. Device for sensing the opening of the eyelids and the initial sleeping phase, according to claims 1, 2, 5, 11 and 17, characteristic in that module (7) additionally incorporates an analogic/digital converter (60), the input of which comes from filter (32) and the output of which is read directly by microcontroller (63).

19. Device for sensing the opening of the eyelids and the initial sleeping phase, according to claims 1, 2, 7, 11 and 17, characteristic in that module (8) additionally incorporates an analogic/digital converter (61), the input of which comes from filter (33) and the output of which is read directly by microcontroller (63).

20. Device for sensing the opening of the eyelids and the initial sleeping phase, according to claims 1, 2, 9, 11 and 17, characteristic in that module (9) additionally incorporates an analogic/digital converter (62), the input of which comes from filter (34) and the output of which is read directly by microcontroller (63).

21. Device for sensing the opening of the eyelids and the initial sleeping phase, according to claims 1, 2, 5, 7, 9, 11 and 17, characteristic in that module (10) is implemented by software and in that input in the initial sleeping phase is indicated by digital output (65) becoming activated and by a square signal being generated at digital output (64), both outputs pertaining to microcontroller (63).

22. Device for sensing the opening of the eyelids and the initial sleeping phase, according to claims 1, 2, 5, 7, 9 and 17 to 21, characteristic in that microcontroller (63) receives signals from modules (7), (8) and (9), establishing the loss of consciousness condition through statistical procedures.

23. Device for sensing the opening of the eyelids and the initial sleeping phase, according to claims 1, 13 and 17 to 22, characteristic in that digital output (64) from said microcontroller (63) is connected to exciter circuit (53), a square signal being generated in said digital output.

24. Device for sensing the opening of the eyelids and the initial sleeping phase, according to claims 11, 12 and 17 to 23, characteristic in that microcontroller (63) has an output (64) connected to an amplifier (58) input.

25. Device for sensing the opening of the eyelids and the initial sleeping phase, according to claims 15 and 17 to 24, characteristic in that microcontroller (63) has a digital output (65) leading to connector (49).
